# EUROPEAN PATENT SPECIFICATION

(11) **EP 3 144 018 B1**
(45) Date of publication and mention of the grant of the patent: **26.09.2018**
(21) Application number: 15792801.1
(22) Date of filing: 30.04.2015
(51) Int. Cl.: A61L 31/10, A61L 31/08, A61L 31/02, A61L 31/14

(54) **METHOD FOR PREPARING SURFACE COATING WITH REDUCED DEGRADATION RATE OF BIODEGRADABLE MAGNESIUM ALLOY VASCULAR STENT**
VERFAHREN ZUR HERSTELLUNG EINER OBERFLÄCHENBESCHICHTUNG MIT REDUZIERTER ABBAUGESCHWINDIGKEIT EINES GEFÄSSSTENTS AUS EINER BIOLOGISCH ABBAUBAREN MAGNESIUMLEGIERUNG
PROCÉDÉ DE PRÉPARATION DE REVÊTEMENT DE SURFACE À VITESSE DE DÉGRADATION RÉDUITE DE STENT VASCULAIRE EN ALLIAGE DE MAGNÉSIUM BIODÉGRADABLE

(30) Priority: 13.05.2014 CN 201410200367
(43) Date of publication of application: 22.03.2017
(73) Proprietor: Jiangsu Fengyuan Medical Devices Co., Ltd., Industrial Park Suzhou Jiangsu 215123 (CN)
(72) Inventor: LIU, Jing, Beijing 100067 (CN); WANG, Min, Beijing 100067 (CN); ZHANG, Zhixiong, Beijing 100067 (CN); XI, Tingfel, Fengtai District Beijing 100067 (CN)
(74) Representative: Hanna Moore + Curley
(86) International application number: PCT/CN2015/077943
(87) International publication number: WO 2015/172664

(56) References cited:
- WO-A2-2007/095393
- CN-A- 102 008 751
- CN-A- 102 596 274
- CN-A- 102 657 899
- CN-A- 102 912 335
- CN-A- 104 189 963
- US-A1- 2011 217 351
- Huang Jing-Jing ET AL: "Preparation and property of coating on degradable Mg implant", The Chinese Journal of Nonferrous Metals, September 2007 (2007-09), pages 1465-1469, XP055364902, Retrieved from the Internet: URL:http://www.cjonm.com/down/upfile/soft/ 2008429/2008429103348500.pdf [retrieved on 2017-04-13]
- JINGXIN YANG ET AL: "Surface Modifications of Magnesium Alloys for Biomedical Applications", ANNALS OF BIOMEDICAL ENGINEERING, KLUWER ACADEMIC PUBLISHERS-PLENUM PUBLISHERS, NE, vol. 39, no. 7, 29 March 2011 (2011-03-29) , pages 1857-1871, XP019913620, ISSN: 1573-9686, DOI: 10.1007/S10439-011-0300-Y
- DATABASE WPI Week 201143 Thomson Scientific, London, GB; AN 2011-F52233 XP002769302, & CN 102 008 751 A (BEIJING DAOMIAO HAOBO TECHNOLOGY DEV CO LTD) 13 April 2011 (2011-04-13)
- LIPING XU ET AL: "Characteristics and cytocompatibility of biodegradable polymer film on magnesium by spin coating", COLLOIDS AND SURFACES. B, BIOINTERFACES, vol. 93, 1 May 2012 (2012-05-01), pages 67-74, XP055365006, NL ISSN: 0927-7765, DOI: 10.1016/j.colsurfb.2011.12.009
- DATABASE WPI Week 201377 Thomson Scientific, London, GB; AN 2013-D38616 XP002769303, & CN 102 793 947 A (GUANGZHOU RES INST NONFERROUS METALS) 28 November 2012 (2012-11-28)
- JING LIU ET AL: "Enhanced in Vitro and in Vivo Performance of Mg-Zn-Y-Nd Alloy Achieved with APTES Pretreatment for Drug-Eluting Vascular Stent Application", ACS APPLIED MATERIALS AND INTERFACES, vol. 8, no. 28, 20 July 2016 (2016-07-20), pages 17842-17858, XP055365003, US ISSN: 1944-8244, DOI: 10.1021/acsami.6b05038

## Description

### TECHNICAL FIELD

The present invention relates to a preparation method of magnesium alloy biological materials and in particular to a method for preparing surface coating with reduced degradation rate of a biodegradable magnesium alloy vascular stent, as defined in the claims.

### BACKGROUND OF THE PRESENT INVENTION

The interventional intravascular stent treatment has become the most important means in the cardiovascular disease treatment field in recent decade, and the researches on intravascular stent materials have also undergone three development stages, i.e., permanent bare stents, permanent drug-loaded stents, and biodegradable metal stents and high-polymer stents.

At present, stainless steel, cobalt-chromium alloy, nickel-titanium alloy and other permanent bare stents are commonly used in clinics, and remain in the human body as foreign matters after the treatment, thereby stimulate the vascular wall, significantly increase the intimal hyperplasia rate of blood vessels and possibly result in the restenosis of blood vessels. Meanwhile, polymer stents cannot get access to small blood vessels at the end due to their large thickness and size, have unsatisfactory traceability by X rays, need to be heated upon use, thus, it is inconvenient for application; Besides, they have poor radial support and large resilience so that there are potential hazards to the blood vessels. In contrast, degradable magnesium alloy stents have significant advantages: (1) the degradable magnesium alloy stents are degradable; magnesium has a relatively low standard electrode potential of -2.37 V, and worse corrosion resistance in a human physiological environment containing Cl⁻; after a degradable magnesium alloy stent is implanted to a vasculopathy site according to a specified path, the stent will completely degrade after channeling the blocked blood vessels within a predetermined period of time, and the degradation products will be slowly adsorbed by the normal metabolism of the human body without any foreign matter residual in the blood vessels, and the possibility of restenosis of blood vessels is reduced; (2) as a necessary trace metal element in the human body, magnesium exhibits excellent biocompatibility and histocompatibility, participates in the protein synthesis, and is capable of activating multiple enzymes in the body, regulating the activities of the nerve muscle and the central nervous system as well as guaranteeing the normal contraction of cardiac muscle, so that the ion release at a proper rate will not cause local inflammatory response; and (3) the degradable magnesium alloy stents exhibit excellent mechanical property and human tissue compatibility, magnesium and its alloys have high specific strength and specific stiffness, and the Young modulus is about 41-45 GPa, which is less than half of 110-117 GPa of titanium alloys, so that the stress-shielding effect may be effectively relieved.

Chinese Patent CN201310306991.1 has disclosed a preparation method of a surface coating capable of regulating the degradation rate of a magnesium alloy intravascular stent, wherein the stent substrate is made of an Mg-Nd-Zn-Zr magnesium alloy, the surface of which is coated with a polymer such as polylactic acid containing an alkaline earth metal or other additive particles. The degradation speed of the coating is controlled by regulating the content of the additives, so that the degradation speed of the intravascular stent is controlled. However, the coating exhibits weak bonding with the substrate.

Chinese Patent CN200920089853. 1 has disclosed the composite coating of a cardiovascular drug eluting stent, wherein the stent is manufactured by cutting metal pipes by laser; nano-pores are formed by electrochemical corrosion with an acid solution on the surface of the stent; the surface of the stent body is coated with a layer of non-degradable bioactive coating with a thickness ranging from 10 microns to 50 microns; a layer of degradable biocompatible polymer drug-eluting coating with a thickness ranging from 10 microns to 100 microns is coated on the non-degradable bioactive coating; and the drug-eluting coating is composed of a degradable biocompatible high-molecular polymer and restenosis-resistant drug ingredients. This patent has the advantage of slow drug release and can effectively reduce restenosis and thrombosis at the later stage of stent implantation.

Chinese Patent CN200910245022.3 has disclosed an absorbable magnesium alloy stent with an inorganic/organic corrosion-resistant biocompatible composite coating and a preparation method thereof, wherein a magnesium alloy stent scaffold is made of a magnesium alloy WE4 composed of 3.7-4.3% in mass of Y, 2.4-4.4% in mass of RE (2.0-2.5% of Nd) and more than 0.4% of Zr; a surface layer of the stent scaffold is an inorganic porous corrosion-resistant coating; and a dense organic pore-sealing coating is provided outside the inorganic porous corrosion-resistant coating, a drug eluting coating is provided outside the dense organic pore-sealing coating, and a drug slow-release coating is provided outside the drug eluting coating. The corrosion resistance of the magnesium alloy stent scaffold is effectively improved and the release rate of magnesium ions is controlled effectively. Meanwhile, the organic membrane layer improves the surface biocompatibility. The degradation products have no side effect, can be absorbed by human bodies, and have good biocompatibility and blood compatibility. The drug-eluting coating can reduce the burst release of drug and ensure the sustained slow-release of drug.

Chinese Patent CN200610130594.3 has disclosed an absorbable magnesium alloy stent with double release-controlled coatings and a preparation method thereof. The stent scaffold is made of magnesium alloy WE43 containing yttrium, neodymium, zirconium, ytterbium, erbium or other metal elements. On the surface layer of the scaffold, a dense corrosion-resistant coating of magnesium aluminum oxides or cerium oxides is provided, a crosslinked dense drug-loaded coating composed of chitosan or collagen is provided outside the dense corrosion-resistant coating, a non-crosslinked drug-loaded coating composed of poly-L-lactic acid or polyglycolic acid is provided outside the crosslinked dense drug-loaded coating, and a release-controlled coating composed of poly-L-lactic acid or polyglycolic acid is provided outside the non-crosslinked drug-loaded coating. The stent has the advantage that it is strong in bonding and difficult to peel off since the coatings on the stent are double release-controlled coatings.

As can be seen, in order to solve the deficiencies of the magnesium alloys-as materials for intravascular stents-- too fast in degradation and have a lifespan insufficient to channel blood vessels and promote endothelialization, a common method is to deposit a polymer coating. The adopted polymer is a polyester, for example, polylactic acid, polycaprolactone and the like. However, the direct coating process results in weak bonding of the coating with the magnesium alloy substrate and thus poor protective capability of the coating. Consequently, the coating degrades too prematurely and thus loses its protection function.

At present, most of the research technique is to prepare one or more organic or inorganic intermediate membrane layers between a polymer coating and a magnesium alloy substrate by various methods, so as to achieve the purpose of enhancing the bonding of the polymer coating with the magnesium alloy substrate and reducing the degradation rate of the substrate material.

Therefore, the present invention uses a sliane coupling agent as an intermediate coating, for the purpose of improving the bonding of a polymer coating with a magnesium alloy substrate and playing its physical shielding role in preventing the corrosion of Cl⁻ and other corrosive ions in the body fluid, so as to improve the protection of the coating to the substrate and thus realize the effects of reducing the corrosion rate of the substrate and improving the biocompatibility. Huang Jing-Jing et al. disclose in The Chinese Journal of Nonferrous Metals, 2007, 1465-1469, a process for preparing a poly(lactic acid) coating on a degradable Mg alloy implant, after surface pretreatment with silane coupling agents to improve adhesion strength between the Mg implant and poly(lactic acid). Chinese patent application CN-A-102 008 751 discloses a process for preparing a biodegradable stent, e.g. of a Mg alloy, comprising growing a silane coupling layer on the alloy surface, adding a polymeric transition layer and grafting a degradable polymer functional layer.

### SUMMARY OF THE PRESENT INVENTION

The present teaching provides a method as detailed in claim 1. Advantageous features are provided in dependent claims.

Any subject-matter falling outside the scope of the claims is provided for information purposes only. Given this, a main objective of the present invention is to provide a method for preparing surface coating with reduced degradation rate of biodegradable magnesium alloy vascular stent. By the technical solution, the bonding of the polyester polymer coating with the magnesium alloy substrate is improved, due to the excellent mechanical property, biocompatibility and biodegradability, due to the fact that aminos carried by a special silane coupling agent and hydroxyls formed by hydrolysis can be bonded to a polyester polymer and a magnesium alloy respectively. By the characteristics that the magnesium alloy substrate is alkalized during corrosion, and the polyester polymer is acidulated during degradation, the corrosion rate of the magnesium and the pH value of the surrounding solution are regulated, so that the influence to the surrounding tissues is reduced and the requirements on medical magnesium alloys as cardiovascular stent materials are met.

For this purpose, the technical solution of the present invention is realized as below.

A method for preparing surface coating with reduced degradation rate of biodegradable magnesium alloy vascular stent is provided, including the following steps of:
(1) preparing a silane coupling agent coating: mixing a silane coupling agent with an organic solvent to form a silane coupling grafting agent, grafting the silane coupling grafting agent to a surface of a magnesium alloy for a biodegradable vascular stent by a dip-coating process, heating and curing to form a crosslinked silane coupling agent coating; and
(2) preparing a polymer coating: dissolving and mixing a specific polymer into an organic solvent to form a spin-coating polymer solvent, coating the polymer spin-coating solvent on a sample surface having the silane coupling grafting agent coating formed in the step (1) by a spin-coating process, drying under vacuum and volatilizing the polymer spin-coating solvent to form a polymer coating, wherein the conditions in the dip-coating process are as defined in claim 1.

The silane coupling agent is any one of γ-aminopropyltriethoxysilane KH550, N-(β-aminoethyl)-γ-aminopropyl methyldimethoxysilane KH602 and γ-aminoethyl aminopropyltrimethoxysilane KH792.

The surface of the magnesium alloy for the biodegradable vascular stent is cleaned with a cleaning solvent, which is any one of absolute ethanol, anhydrous ether and deionized water.

The magnesium alloy for the biodegradable intravascular stent is put into the silane coupling grafting agent which is 3.0% to 10.0% in volume (v/v), the dip-coating lasts for 30 min to 60 min, the temperature for heating and curing is 60 °C to 120 °C, and the curing lasts for 60 min to 120 min.

A biodegradable polymer material in the polymer is any one of polylactic acid, polycaprolactone, poly(trimethylene carbonate, polylactic acid-trimethylene carbonate copolymer, polycaprolactone-trimethylene carbonate copolymer, polyglycolic acid, polyactic acid-glycolic acid copolymer.

The organic solvent is any one of dichloromethane, trichloromethane, methanol, absolute ethanol and acetone, which is 1.0% to 4.0% in mass (w/v).

In the step (2), the rotation speed for spin-coating is 200 r/min to 6000 r/min, and the spin-coating lasts for 6 s to 20 s.

In the step (2), the temperature for vacuum drying is 37 °C, and the vacuum drying lasts for 48 h to 60 h.

The thickness of the polymer coating formed in the step (2) is 20 µm to 100 µm.

The magnesium alloy for the biodegradable vascular stent is any one of Mg-RE alloys, WE alloys, AZ alloys, AM alloys, ZK alloys, ZM alloys, Mg-Li alloys and Mg-Ca alloys.

The technical solution has the following beneficial effects: a preparation method of a surface coating for reducing the degradation rate of biodegradable magnesium alloy vascular stent is provided; by the technical solution, first, in comparison with the directly coated polymer coating, the magnesium alloy polymer coating pre-treated by the silane coupling agent may form a chemical bond between the polymer coating and the magnesium alloy substrate, so that the influence of the in-vitro and in-vivo environments to the magnesium alloy substrate is significantly impeded, the corrosion rate of the magnesium alloy substrate in the in-vitro and in-vivo environments is greatly reduced, and the peeling off from surface of the magnesium alloy for the intravascular stent may be effectively relieved; second, for the magnesium alloy polymer coating pre-treated by the silane coupling agent, the silane coupling agent is alkaline after degradation so that the silane coupling agent can neutralize the acid microenvironment caused by the degradation of the coating and inhibit the degradation of the substrate; moreover, as the content of the silane coupling agent is very low, the silane coupling agent will completely degrade in the human body together with the substrate and the polymer coating, without any side effect on the human body; third, for the magnesium alloy polymer coating pre-treated by the silane coupling agent, the degree of crosslinking of the magnesium alloy polymer coating with the magnesium alloy surface and the polymer may be controlled by limiting the amount of the silane coupling agent, so that the degradation rate of the polymer and the silane coupling agent coating is controlled and the degradation rate of the magnesium alloy for the intravascular stent is thus controlled; and, fourth, this technical solution is easy to operate, efficient and environmentally-friendly.

### BRIEF DESCRIPTION OF THE DRAWINGS

Fig. 1 is an electrochemical open circuit potential (OCP) curve of MgZnYNd, 2% PLGA-MgZnYNd and 2% PLGA-KH550-MgZnYNd according to an embodiment of the present invention;
Fig. 2 is an electrochemical impedance spectroscopy (EIS) curve of MgZnYNd, 2% PLGA-MgZnYNd and 2% PLGA-KH550-MgZnYNd according to an embodiment of the present invention;
Fig. 3 is an electrochemical polarization Tafel curve of MgZnYNd, 2% PLGA-MgZnYNd and 2% PLGA-KH550-MgZnYNd according to an embodiment of the present invention;
Fig. 4 is an SEM images of (a) MgZnYNd, (b) 2% PLGA-MgZnYNd and (c) 2% PLGA-KH550-MgZnYNd after immersion for 30 days according to an embodiment of the present in vention;
Fig. 5 is an EDS graph of (a) MgZnYNd after immersion for 30 days according to an embodiment of the present invention;
Fig. 6 is an EDS graph of (b) 2% PLGA-MgZnYNd after immersion for 30 days according to an embodiment of the present invention;
Fig. 7 is an EDS graph of (c) 2% PLGA-KH550-MgZnYNd after immersion for 30 days according to an embodiment of the present invention;
Fig. 8 is an SEM images of VSMC and ECV304 cell adhesion of MgZnYNd, 2% PLGA-MgZnYNd and 2% PLGA-KH550-MgZnYNd according to an embodiment of the present invention;
Fig. 9 shows a (a) VSMC cytotoxicity experiment of MgZnYNd, 2% PLGA-MgZnYNd and 2% PLGA-KH550-MgZnYNd according to an embodiment of the present invention, with a incubation time of 1 day, 3 days and 5 days; and
Fig. 10 shows a (b) ECV304 cytotoxicity experiment of MgZnYNd, 2% PLGA-MgZnYNd and 2% PLGA-KH550-MgZnYNd according to an embodiment of the present invention, with incubation time of 1 day, 3 days and 5 days.

### DETAILED DESCRIPTION OF THE PRESENT INVENTION

The technical solution of the present invention will be further described below in details with reference to the accompanying drawings and specific embodiments.

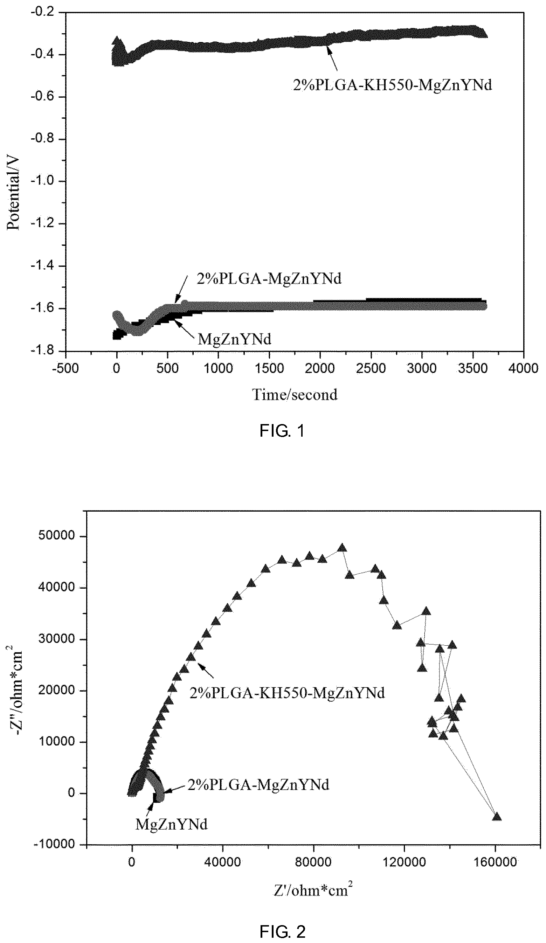

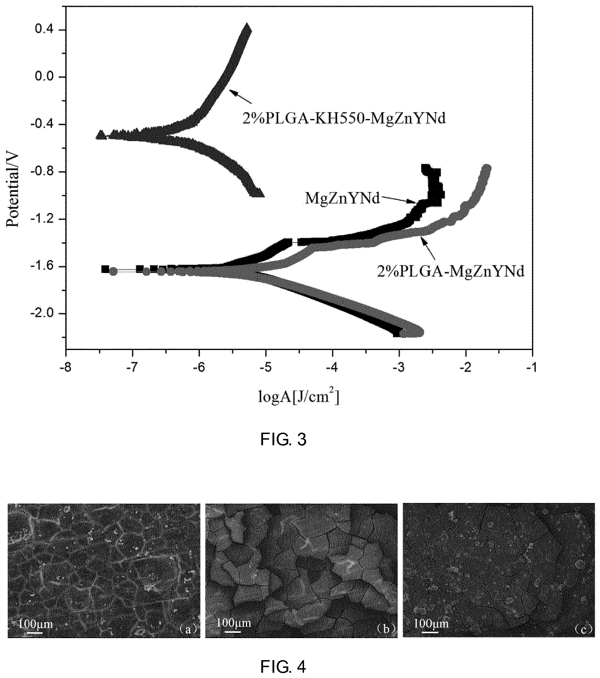

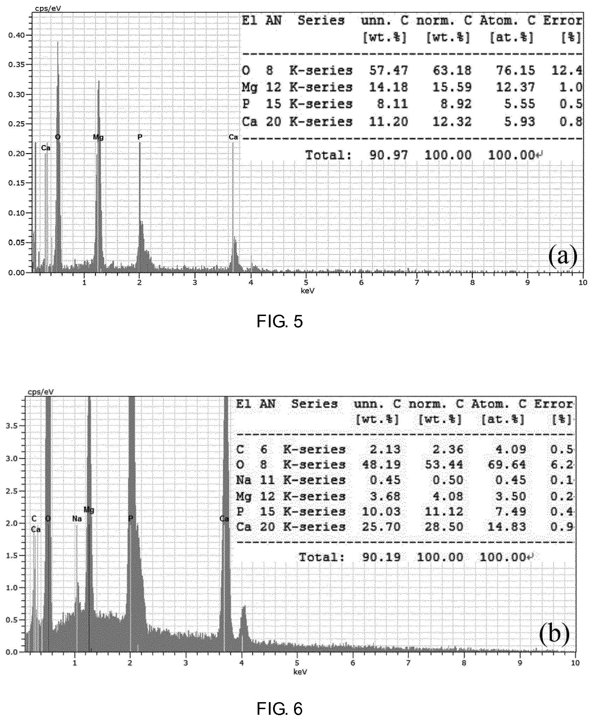

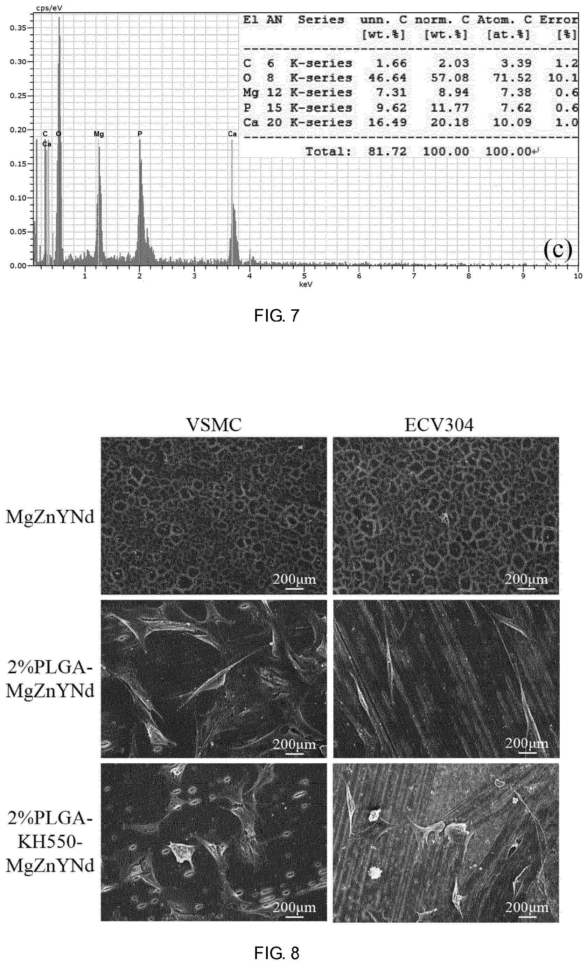

Reference will be made to Figs. 1-8.

The present invention relates to a preparation method of a surface coating for reducing the degradation rate of a completely-degradable magnesium alloy intravascular stent. The method is performed by the following steps of:
(1) preparing a silane coupling agent coating: mixing a silane coupling agent with an organic solvent to form a silane coupling grafting agent, grafting the silane coupling grafting agent to a surface of a magnesium alloy for a biodegradable vascular stent by a dip-coating process, heating and curing to form a crosslinked silane coupling agent coating; and
(2) preparing a polymer coating: dissolving and mixing a specific polymer into an organic solvent to form a polymer spin-coating solvent, coating the polymer spin-coating solvent on a sample surface having the silane coupling grafting agent coating formed in the step (1) by a spin-coating process, drying under vacuum and volatilizing the polymer spin-coating solvent to form a polymer coating, wherein the conditions of the dip-coating process are as defined in claim 1.

Preferably, the silane coupling agent is any one of γ-aminopropyltriethoxysilane KH550, N-(β-aminoethyl)-γ-aminopropyl methyldimethoxysilane KH602 and γ-aminoethyl aminopropyltrimethoxysilane KH792.

As a further technical solution, the surface of the magnesium alloy for the biodegradable intravascular stent is cleaned with a cleaning solvent which is any one of absolute ethanol, anhydrous ether and deionized water.

According to the invention, the magnesium alloy for the biodegradable intravascular stent is put into the silane coupling grafting agent which is 3.0% to 10.0% in volume (v/v), the dip-coating lasts for 30 min to 60 min, the temperature for heating and curing is 60°C to 120°C, and the curing lasts for 60 min to 120 min. In the present invention, a biodegradable polymer material in the polymer is any one of polylactic acid, polycaprolactone, poly(trimethylene carbonate), polylactic acid-trimethylene carbonate copolymer, polycaprolactone-trimethylene carbonate copolymer, polyglycolic acid, polyactic acid-glycolic acid copolymer.

In the present invention, the organic solvent is any one of dichloromethane, trichloromethane, methanol, absolute ethanol and acetone, which is 1.0% to 4.0% in mass (w/v).

As a further technical solution, in the step (2), the rotation speed for spin-coating is 200 r/min to 6000 r/min, and the spin-coating lasts for 6 s to 20 s.

In the present invention, in the step (2), the temperature for vacuum drying is 37°C, and the vacuum drying lasts for 48h to 60h.

Preferably, the thickness of the polymer coating formed in the step (2) is 20 µm to 100 µm.

In the present invention, the magnesium alloy for the biodegradable vascular stent is any one of Mg-RE alloys, WE alloys, AZ alloys, AM alloys, ZK alloys, ZM alloys, Mg-Li alloys and Mg-Ca alloys.

### Embodiment 1

In Embodiment 1 of the preparation method of a surface coating for reducing the degradation rate of biodegradable magnesium alloy vascular stent provided by the present invention, the magnesium alloy for the biodegradablevascular stent is Mg-Zn-Y-Nd, the silane coupling agent is γ-aminopropyltriethoxysilane KH550, the specific polymer is a polyactic acid-glycolic acid particle solution having a relative molecular mass of 100,000 (LA:PA=75:25), and the organic solvent is a dichloromethane solution. The method includes the following steps.

Step 1: Preparation of a silane coupling agent coating: the silane coupling agent γ-aminopropyltriethoxysilane KH550 was dissolved in the organic solvent dichloromethane to form a dichloromethane solution of a silane coupling grafting agent KH550 which is 5% in volume (v/v), and the solution was stirred for 0.5 h; the biodegradable Mg-Zn-Y-Nd magnesium alloy was linearly cut and processed into sheets with a diameter ø of 10 mm and a wall thickness d of 0.8 mm, and the sheets were polished to 2000^{#}, then washed with acetone and deionized water, and ultrasonically cleaned for 30 min in absolute ethanol; the samples were dipped in the 5% (v/v) silane coupling grafting agent KH550 solution for 0.5 h, then grafted to the surface of the Mg-Zn-Y-Nd magnesium alloy and heated for curing for 100 min at 60°C to form a crosslinked silane coupling agent layer.

Step 2: Preparation of a polymer coating: polyactic acid-glycolic acid particles having a relative molecular mass of 100,000 (LA:PA=75:25) were dissolved in dichloromethane to form a PLGA solution which is 2% in mass (w/v); the polymer was coated on the surfaces of the samples treated by the silane coupling grafting agent by a spin-coating process, held for 6 s at 200 rpm and for 20s at 6000 rpm; and the samples were dried for 48 h in a vacuum drying oven at 37 °C to volatilize the solvent so as to form a polyactic acid-glycolic acid coating.

As shown in Figs. 1 to 10, the experiments indicate that, by comparing the product prepared in Embodiment 1 with the Mg-Zn-Y-Nd magnesium alloy that is not pre-treated by the silane coupling grafting agent KH550, the EIS curve in an electrochemical experiment showed a significantly larger capacitive loop diameter, the corrosion potential E_{corr} obtained by fitting Tafel polarization curves increased from -1.644 V to -0.499 V, and the corrosion current I_{corr} reduced from 5.192 µA·cm² to 1.339 µA·cm²; the average degradation rate in the Hank's simulated body fluid reduced from 0.12 mg/(cm²·day) to 0.02 mg/(cm²·day); in a cytotoxicity experiment, the survival rate of smooth muscle cells (VSMCs) reduced by about 10% within 1 to 3 days, the survival rate of endothelial cells ECV304 increased by about 3% to 50% within 1 to 5 days. Thus, it is indicated that there are potentials to facilitate the healing of endothelial cells and prevent the restenosis of blood vessels. In addition, the number of both of the cells (VSMCs and ECV304) adhered to the surface of the material treated by the silane coupling agent was much higher than that of the untreated group.

### Embodiment 2

In Embodiment 2 of the preparation method of a surface coating for reducing the degradation rate of biodegradable magnesium alloy vascular stent provided by the present invention, the method includes the following steps.

Step 1: Preparation of a silane coupling agent coating: the silane coupling agent γ-aminopropyltriethoxysilane KH550 was dissolved in dichloromethane to form a dichloromethane solution of a silane coupling grafting agent KH550 which is 5% in volume (v/v), and the solution was stirred for 0.5 h; the biodegradable magnesium alloy was linearly cut and processed into sheets with a diameter ø of 10 mm and a wall thickness d of 0.8 mm, and the sheets were polished to 2000^{#}, then washed with acetone and deionized water, and ultrasonically cleaned for 30 min in absolute ethanol; the samples were dipped in the 5% (v/v) silane coupling grafting agent KH550 solution for 1.0 h, and the silane coupling grafting agent KH550 was grafted to the surface of the magnesium alloy and heated for curing for 60 min at 100°C to form a crosslinked silane coupling agent layer.

Step 2: Preparation of a polymer coating: polyactic acid-glycolic acid particles having a relative molecular mass of 100,000 (LA:PA=75:25) were dissolved in dichloromethane to form a PLGA solution which is 4% in mass (w/v); the polymer was coated on the surfaces of the samples treated by the silane coupling grafting agent by a spin-coating process, held for 6 s at 200 rpm and for 20 s at 8000 rpm; and, the samples were dried for 60 h in a vacuum drying oven at 37 °C to volatilize the solvent so as to form a polyactic acid-glycolic acid coating.

Implementation effects: by comparing the product prepared in Embodiment 2 with the magnesium alloy that was not pre-treated by the silane coupling grafting agent KH550, the EIS curve in an electrochemical experiment showed a significantly increased capacitive loop diameter, the corrosion potential E_{corr} obtained by fitting Tafel polarization curves increased from -0.401 V to -0.259 V, and the corrosion current I_{corr} reduced from 1.130 µA·cm² to 0.141 µA·cm²; the average degradation rate in the Hank's simulated body fluid reduced from 0.12 mg/(cm²·day) to 0.04 mg/(cm²·day); in a cytotoxicity experiment, the survival rate of smooth muscle cells (VSMCs) reduced by about 10% to 25% within 1 to 3 days, the survival rate of endothelial cells ECV304 increased by about 12% to 45% within 1 to 5 days. Thus, it is indicated that there are potentials to facilitate the healing of endothelial cells and prevent the restenosis of blood vessels. In addition, the number of both of the cells (VSMCs and ECV304) adhered to the surface of the material treated by the silane coupling agent was much higher than that of the untreated group.

### Embodiment 3

In Embodiment 3 of the preparation method of a surface coating for reducing the degradation rate of biodegradable magnesium alloy vascular stent provided by the present invention, the method includes the following steps.

Step 1: Preparation of a silane coupling agent coating: the silane coupling agent γ-aminopropyltriethoxysilane KH550 was dissolved in dichloromethane to form a dichloromethane solution of a silane coupling grafting agent KH550 which is 3% in volume (v/v), and the solution was stirred for 0.5 h; the biodegradable magnesium alloy was linearly cut and processed into sheets with a diameter ø of 10 mm and a wall thickness d of 0.8 mm, and the sheets were polished to 2000^{#}, then washed with acetone and deionized water and ultrasonically cleaned for 30 min in absolute ethanol; the samples were dipped in the 3% (v/v) silane coupling grafting agent KH550 solution for 1.0 h, and the silane coupling grafting agent KH550 was grafted to the surface of the magnesium alloy and heated for curing for 60 min at 60 °C to form a crosslinked silane coupling agent layer.

Step 2: Preparation of a polymer coating: polyactic acid-glycolic acid particles having a relative molecular mass of 100,000 (LA:PA=75:25) were dissolved in dichloromethane to form a PLGA solution which is 1% in mass (w/v); the polymer was coated on the surfaces of the samples treated by the silane coupling grafting agent by a spin-coating process, held for 6 s at 200 rpm and for 20 s at 4000 rpm; and, the samples were dried for 60 h in a vacuum drying oven at 37 °C to volatilize the solvent so as to form a polyactic acid-glycolic acid coating.

Implementation effects: by comparing the product prepared in Embodiment 3 with the magnesium alloy that was not pre-treated by the silane coupling grafting agent KH550, the EIS curve in an electrochemical experiment showed a significantly increased capacitive loop diameter , the corrosion potential E_{corr} obtained by fitting Tafel polarization curves increased from -1.614V to -0.625V, and the corrosion current I_{corr} reduced from 5.540 µA·cm² to 0.734 µA·cm²; the average degradation rate in the Hank's simulated body fluid reduced from 0.15 mg/(cm²·day) to 0.07mg/(cm²·day); in a cytotoxicity experiment, the survival rate of smooth muscle cells (VSMCs) reduced by about 4% to 8% within 1 to 3 days, the survival rate of endothelial cells ECV304 increased by about 3% to 20% within 1 to 5 days. Thus, it is indicated that there are potentials to facilitate the healing of endothelial cells and prevent the restenosis of blood vessels. In addition, the number of both of the cells (VSMCs and ECV304) adhered to the surface of the material treated by the silane coupling agent was much higher than that of the untreated group.

### Embodiment 4

In Embodiment 4 of the preparation method of a surface coating for reducing the degradation rate of biodegradable magnesium alloy vascular stent provided by the present invention, the method includes the following steps.

Step 1: Preparation of a silane coupling agent coating: the silane coupling agent N-(β-aminoethyl)-γ-aminopropyl methyldimethoxysilane KH602 was dissolved in absolute ethanol to form an ethanol solution of a silane coupling grafting agent KH602 which is 5% in volume (v/v), and the solution was stirred for 1h; the biodegradable magnesium alloy for the intravascular stent was linearly cut and processed into sheets with a diameter ø of 10 mm and a wall thickness d of 0.8 mm, and the sheets were polished to 2000^{#} and then ultrasonically cleaned for 30 min in absolute ethanol; the samples were dipped in the 5% (v/v) silane coupling grafting agent KH602 solution for 0.5 h, and the silane coupling grafting agent KH602 was grafted to the surface of the magnesium alloy and heated for curing for 40 min at 75 °C to form a crosslinked silane coupling agent layer.

Step 2: Preparation of a polymer coating: polyglycolic acid particles having a relative molecular mass of 150,000 were dissolved in dichloromethane to form a PGA solution which is 4% in mass (w/v); the polymer was coated on the surfaces of the samples treated by the silane coupling grafting agent by a spin-coating process, held for 6s at 200 rpm and for 20 s at 8000 rpm; and, the samples were dried for 48 h in a vacuum drying oven at 37 °C to volatilize the solvent so as to form a polyglycolic acid coating.

Implementation effects: by comparing the product prepared in Embodiment 4 with the magnesium alloy that was not pre-treated by the silane coupling grafting agent KH602, the corrosion potential E_{corr} obtained by fitting Tafel polarization curves obviously increased, and the corrosion current I_{corr} significantly reduced; the EIS curve showed a significantly increased capacitive loop diameter; the average degradation rate in the Hank's simulated body fluid significantly reduced; and the survival rate and the number of both of the cells (VSMCs and ECV304) adhered increased correspondingly.

### Embodiment 5

In Embodiment 5 of the preparation method of a surface coating for reducing the degradation rate of a biodegradable magnesium alloy vascular stent provided by the present invention, the method includes the following steps.

Step 1: Preparation of a silane coupling agent coating: the silane coupling agent N-(β-aminoethyl)-γ-aminopropyl methyldimethoxysilane KH792 was dissolved in absolute ethanol to form an ethanol solution of a silane coupling grafting agent KH792 which is 5% in volume (v/v), and the solution was stirred for 1 h; the biodegradable magnesium alloy for the intravascular stent was linearly cut and processed into sheets with a diameter ø of 10 mm and a wall thickness d of 0.8 mm, and the sheets were polished to 2000^{#} and then ultrasonically cleaned for 30 min in absolute ethanol; the samples were dipped in the 5% (v/v) silane coupling grafting agent KH792 solution for 0.5 h, and the silane coupling grafting agent KH792 was grafted to the surface of the magnesium alloy and heated for curing for 30 min at 120 °C to form a crosslinked silane coupling agent layer.

Step 2: Preparation of a polymer coating: polylactic acid particles having a relative molecular mass of 80,000 were dissolved in dichloromethane to form a PLA solution which is 4% in mass (w/v); the polymer was coated on the surfaces of the samples treated by the silane coupling grafting agent by a spin-coating process, held for 6 s at 200 rpm and for 20 s at 6000 rpm; and, the samples were dried for 60 h in a vacuum drying oven at 37 °C to volatilize the solvent so as to form a polylactic acid coating.

Implementation effects: by comparing the product prepared in Embodiment 5 with the magnesium alloy that was not pre-treated by the silane coupling grafting agent KH792, the corrosion potential E_{corr} obtained by fitting Tafel polarization curves obviously increased, and the corrosion current I_{corr} significantly reduced; the EIS curve showed a significantly increased capacitive loop diameter; the average degradation rate in the Hank's simulated body fluid significantly reduced; and the survival rate and the number of both of the cells (VSMCs and ECV304) adhered increased correspondingly.

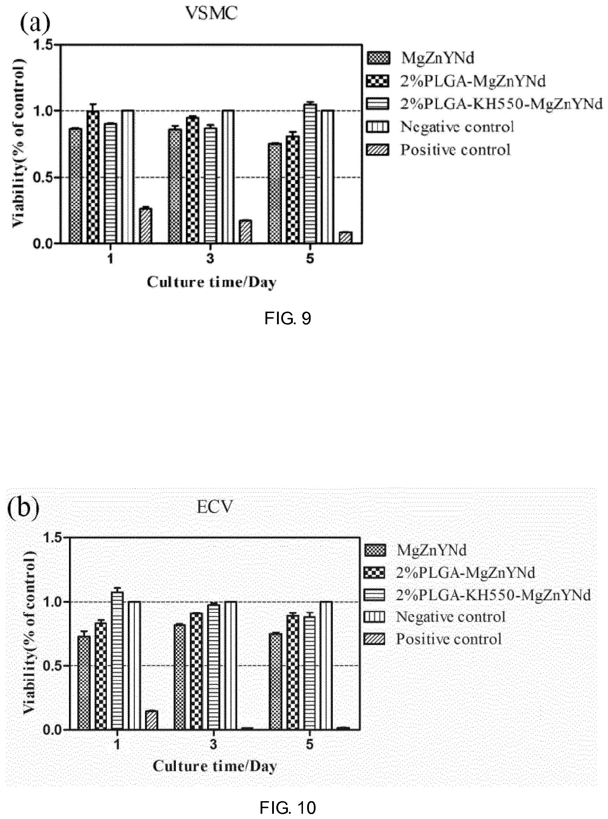

In Figs. 9 and 10 of the present invention, the negative control is a DMEM culture medium containing serum, and the positive control is 10% DMSO.

In conclusion, the magnesium alloy polymer coating, pre-treated by the silane coupling agent, prepared by the method of the present invention can eventually reduce the degradation rate of the magnesium alloy for the biodegradable vascular stent and improve the biocompatibility. The method provided by the present invention is simple, remarkable in effects, efficient and environmentally-friendly, and has good application prospect.

## Claims

1. A method for preparing surface coating with reduced degradation rate of biodegradable magnesium alloy vascular stent, comprising the following steps of:
(1) preparing a silane coupling agent coating: mixing a silane coupling agent with an organic solvent to form a silane coupling grafting agent, grafting the silane coupling grafting agent to a surface of a magnesium alloy for a biodegradable intravascular stent by a dip-coating process; heating and curing to form a crosslinked silane coupling agent coating; and
(2) preparing a polymer coating: dissolving and mixing a specific polymer into an organic solvent to form a polymer spin-coating solvent, coating the polymer spin-coating solvent on a sample surface having the silane coupling grafting agent coating formed in the step (1) by a spin-coating process, drying in vacuum and volatilizing the polymer spin-coating solvent to form a polymer coating;
wherein in the dip-coating process, the magnesium alloy for the biodegradable intravascular stent is put into the silane coupling grafting agent which is 3.0% to 10.0% in volume (v/v), the dip-coating lasts for 30 min to 60 min; and the temperature for heating and curing is 60 °C to 120 °C, and the curing lasts for 60 min to 120 min.

2. The preparation method of a surface coating for reducing the degradation rate of biodegradable magnesium alloy vascular stent according to claim 1, **characterized in that** the silane coupling agent is any one of γ-aminopropyltriethoxysilane KH550, N-(β-aminoethyl)-γ-aminopropyl methyldimethoxysilane KH602 and γ-aminoethyl aminopropyltrimethoxysilane KH792.

3. The preparation method of a surface coating for reducing the degradation rate of biodegradable magnesium alloy vascular stent according to claim 1, **characterized in that** the surface of the magnesium alloy for the biodegradable intravascular stent is cleaned with a cleaning solvent which is any one of absolute ethanol, anhydrous ether and deionized water.

4. The preparation method of a surface coating for reducing the degradation rate of biodegradable magnesium alloy vascular stent according to claim 1, **characterized in that** a biodegradable polymer material of the specific polymer is any one of polylactic acid, polycaprolactone, poly(trimethylene carbonate), polylactic acid-trimethylene carbonate copolymer, polycaprolactone-trimethylene carbonate copolymer, polyglycolic acid, polyactic acid-glycolic acid copolymer.

5. The preparation method of a surface coating for reducing the degradation rate of biodegradable magnesium alloy vascular stent according to claim 1, **characterized in that** the organic solvent is any one of dichloromethane, trichloromethane, methanol, absolute ethanol and acetone, which is 1.0% to 4.0% in mass (w/v).

6. The preparation method of a surface coating for reducing the degradation rate of biodegradable magnesium alloy vascular stent according to claim 1, **characterized in that**, in the step (2), the rotation speed for spin-coating is 200 r/min to 6000 r/min, and the spin-coating lasts for 6 s to 20 s.

7. The preparation method of a surface coating for reducing the degradation rate of a completely-degradable magnesium alloy intravascular stent according to claim 1, **characterized in that**, in the step (2), the temperature for vacuum drying is 37 °C, and the vacuum drying lasts for 48 h to 60 h.

8. The preparation method of a surface coating for reducing the degradation rate of biodegradable magnesium alloy vascular stent according to claim 1, **characterized in that** the thickness of the polymer coating formed in the step (2) is 20 µm to 100 µm.

9. The preparation method of a surface coating for reducing the degradation rate of biodegradable magnesium alloy vascular stent according to claim 1, **characterized in that** the magnesium alloy for the biodegradable vascular stent is any one of Mg-RE alloys, WE alloys, AZ alloys, AM alloys, ZK alloys, ZM alloys, Mg-Li alloys and Mg-Ca alloys.

## Patentansprüche

1. Verfahren zum Vorbereiten einer Oberflächenbeschichtung mit reduzierter Abbaugeschwindigkeit eines Gefäßstents aus biologisch abbaubarer Magnesiumlegierung, die folgenden Schritte umfassend:
(1) Vorbereiten einer Beschichtung aus Silanhaftvermittler: Mischen eines Silanhaftvermittlers mit einem organischen Lösemittel, um ein Silanhaftvermittler-Pfropfmittel auszubilden, Pfropfen des Silanhaftvermittler-Pfropfmittel auf eine Oberfläche einer Magnesiumlegierung für einen biologisch abbaubaren intravasalen Stent in einem Tauchbeschichtungsprozess; Erhitzen und Härten, um eine vernetzte Beschichtung aus Silanhaftvermittler auszubilden; und
(2) Vorbereiten einer Polymerbeschichtung: Lösen und Mischen eines bestimmten Polymers zu einem organischen Lösemittel, um ein polymeres Rotationsbeschichtungslösemittel auszubilden, Beschichten einer Probeoberfläche, die das in Schritt (1) ausgebildete Silanhaftvermittler-Pfropfmittel aufweist, mit dem polymeren Rotationsbeschichtungslösemittel anhand eines Rotationsbeschichtungsprozesses, Trocknen im Vakuum und Verflüchtigen des polymeren Rotationsbeschichtungslösemittels, um eine polymere Beschichtung auszubilden;
wobei im Tauchbeschichtungsprozess die Magnesiumlegierung für den biologisch abbaubaren intravasalen Stent in das Silanhaftvermittler-Pfropfmittel gebracht wird, das 3,0 bis 10,0 Volumenprozent (Vol.-%) ist, die Tauchbeschichtung 30 bis 60 Minuten dauert; und die Temperatur zum Erhitzen und Härten 60 °C bis 120 °C beträgt und das Härten 60 bis 120 Minuten dauert.

2. Vorbereitungsverfahren einer Oberflächenbeschichtung zum Reduzieren der Abbaugeschwindigkeit eines Gefäßstents aus biologisch abbaubarer Magnesiumlegierung nach Anspruch 1, **dadurch gekennzeichnet, dass** der Silanhaftvermittler ein beliebiges aus γ-Aminopropyltriethoxysilan KH550, N-(β-Aminoethyl)-γ-aminopropyl-methyldimethoxysilan KH602 und γ-Aminoethyl-aminopropyltrimethoxysilan KH792 ist.

3. Vorbereitungsverfahren einer Oberflächenbeschichtung zum Reduzieren der Abbaugeschwindigkeit eines Gefäßstents aus biologisch abbaubarer Magnesiumlegierung nach Anspruch 1, **dadurch gekennzeichnet, dass** die Oberfläche der Magnesiumlegierung für den biologisch abbaubaren intravasalen Stent mit einer Reinigungslösung gereinigt wird, die eine beliebige aus reinem Ethanol, wasserfreiem Ether und deionisiertem Wasser ist.

4. Vorbereitungsverfahren einer Oberflächenbeschichtung zum Reduzieren der Abbaugeschwindigkeit eines Gefäßstents aus biologisch abbaubarer Magnesiumlegierung nach Anspruch 1, **dadurch gekennzeichnet, dass** ein biologisch abbaubares polymeres Material des bestimmten Polymers ein beliebiges aus Polylactid, Polycaprolacton, Poly(trimethylencarbonat), Polylactid-Trimethylencarbonat-Copolymer, Polycaprolacton-Trimethylencarbonat-Copolymer, Polyglycolsäure, Polyglycolsäure-Glycolsäure-Copolymer ist.

5. Vorbereitungsverfahren einer Oberflächenbeschichtung zum Reduzieren der Abbaugeschwindigkeit Gefäßstents aus biologisch abbaubarer Magnesiumlegierung nach Anspruch 1, **dadurch gekennzeichnet, dass** das organische Lösemittel ein beliebiges aus Dichlormethan, Trichlormethan, Methanol, reinem Ethanol und Aceton ist, das 1,0 bis 4,0 Massen-% (MA.-%) ist.

6. Vorbereitungsverfahren einer Oberflächenbeschichtung zum Reduzieren der Abbaugeschwindigkeit eines Gefäßstents aus biologisch abbaubarer Magnesiumlegierung nach Anspruch 1, **dadurch gekennzeichnet, dass** in Schritt (2) die Drehzahl für die Rotationsbeschichtung 200 U/min bis 6000 U/min beträgt und das Rotationsbeschichten 6 s bis 20 s dauert.

7. Vorbereitungsverfahren einer Oberflächenbeschichtung zum Reduzieren der Abbaugeschwindigkeit eines vollständig abbaubaren intravasalen Stent aus biologisch abbaubarer Magnesiumlegierung nach Anspruch 1, **dadurch gekennzeichnet, dass** in Schritt (2) die Temperatur zum Vakuumtrocknen 37 °C beträgt und das Vakuumtrocknen 48 Std. bis 60 Std. dauert.

8. Vorbereitungsverfahren einer Oberflächenbeschichtung zum Reduzieren der Abbaugeschwindigkeit eines Gefäßstents aus biologisch abbaubarer Magnesiumlegierung nach Anspruch 1, **dadurch gekennzeichnet, dass** die Dicke der in Schritt (2) ausgebildeten polymeren Beschichtung 20 µm bis 100 µm beträgt.

9. Vorbereitungsverfahren einer Oberflächenbeschichtung zum Reduzieren der Abbaugeschwindigkeit eines intravasalen Stents aus biologisch abbaubarer Magnesiumlegierung nach Anspruch 1, **dadurch gekennzeichnet, dass** die Magnesiumlegierung für den biologisch abbaubaren Gefäßstent eine beliebige aus Mg-RE-Legierungen, WE-Legierungen, AZ-Legierungen, AM-Legierungen, ZK-Legierungen, ZM-Legierungen, Mg-Li-Legierungen und Mg-Ca-Legierungen ist.

## Revendications

1. Procédé de préparation d'un revêtement de surface à vitesse de dégradation réduite d'un stent vasculaire en alliage de magnésium biodégradable, comprenant les étapes suivantes consistant à :
(1) préparer un revêtement d'agent de couplage silane : mélanger un agent de couplage silane avec un solvant organique pour former un agent de greffage de couplage silane, greffer l'agent de greffe de couplage silane sur une surface d'un alliage de magnésium pour un stent intravasculaire biodégradable par un traitement de revêtement par immersion ; chauffer et faire durcir pour former un revêtement d'agent de couplage silane réticulé ; et
(2) préparer un revêtement de polymère : dissoudre et mélanger un polymère spécifique dans un solvant organique pour former un solvant de revêtement par centrifugation de polymère, appliquer en revêtement le solvant de revêtement par centrifugation de polymère sur une surface d'échantillon ayant le revêtement d'agent de greffage de couplage silane formé dans l'étape (1) par un procédé de revêtement par centrifugation, sécher sous vide et volatiliser le solvant de revêtement par centrifugation de polymère pour former un revêtement de polymère ;
où, dans le procédé de revêtement par immersion, l'alliage de magnésium pour le stent intravasculaire biodégradable est mis dans l'agent de greffage de couplage silane qui représente 3,0 % à 10,0 % en volume (v/v), le revêtement par immersion dure 30 min à 60 min ; et la température pour le chauffage et le durcissement est 60°C à 120°C, et le durcissement dure 60 min à 120 min.

2. Procédé de préparation d'un revêtement de surface pour réduire la vitesse de dégradation d'un stent vasculaire en alliage de magnésium biodégradable, selon la revendication 1, **caractérisé par le fait que** l'agent de couplage silane est l'un quelconque parmi le γ-aminopropyltriéthoxysilane KH550, le N-(β-aminoéthyl)-γ-aminopropyl méthyldiméthoxysilane KH602 et le γ-aminoéthyl aminopropyltriméthoxysilane KH792.

3. Procédé de préparation d'un revêtement de surface pour réduire la vitesse de dégradation d'un stent vasculaire en alliage de magnésium biodégradable, selon la revendication 1, **caractérisé par le fait que** la surface de l'alliage de magnésium pour le stent intravasculaire biodégradable est nettoyée avec un solvant de nettoyage qui est l'un quelconque parmi l'éthanol absolu, l'éther anhydre et l'eau désionisée.

4. Procédé de préparation d'un revêtement de surface pour réduire la vitesse de dégradation d'un stent vasculaire en alliage de magnésium biodégradable, selon la revendication 1, **caractérisé par le fait qu'**une matière polymère biodégradable du polymère spécifique est l'une quelconque parmi l'acide polylactique, la polycaprolactone, le poly(carbonate de triméthylène), un copolymère acide polylactique - carbonate de triméthylène, un copolymère polycaprolactone - carbonate de triméthylène, l'acide polyglycolique, un copolymère acide polylactique - acide glycolique.

5. Procédé de préparation d'un revêtement de surface pour réduire la vitesse de dégradation d'un stent vasculaire en alliage de magnésium biodégradable, selon la revendication 1, **caractérisé par le fait que** le solvant organique est l'un quelconque parmi le dichlorométhane, le trichlorométhane, le méthanol, l'éthanol absolu et l'acétone, lequel représente 1,0 % à 4,0 % en masse (p/v).

6. Procédé de préparation d'un revêtement de surface pour réduire la vitesse de dégradation d'un stent vasculaire en alliage de magnésium biodégradable, selon la revendication 1, **caractérisé par le fait que**, dans l'étape (2), la vitesse de rotation pour le revêtement par centrifugation est 200 t/min à 6000 t/min, et le revêtement par centrifugation dure 6 s à 20 s.

7. Procédé de préparation d'un revêtement de surface pour réduire la vitesse de dégradation d'un stent intravasculaire en alliage de magnésium entièrement dégradable, selon la revendication 1, **caractérisé par le fait que**, dans l'étape (2), la température pour le séchage sous vide est 37 °C, et le séchage sous vide dure 48 h à 60 h.

8. Procédé de préparation d'un revêtement de surface pour réduire la vitesse de dégradation d'un stent vasculaire en alliage de magnésium biodégradable, selon la revendication 1, **caractérisé par le fait que** l'épaisseur du revêtement de polymère formé dans l'étape (2) est 20 µm à 100 µm.

9. Procédé de préparation d'un revêtement de surface pour réduire la vitesse de dégradation d'un stent vasculaire en alliage de magnésium biodégradable, selon la revendication 1, **caractérisé par le fait que** l'alliage de magnésium pour le stent vasculaire biodégradable est l'un quelconque parmi les alliages Mg-RE, les alliages WE, les alliages AZ, les alliages AM, les alliages ZK, les alliages ZM, les alliages Mg-Li et les alliages Mg-Ca.
